# EUROPEAN PATENT SPECIFICATION

(11) **EP 0 464 218 B1**
(45) Date of publication and mention of the grant of the patent: **23.08.1995**
(21) Application number: 91902739.1
(22) Date of filing: 22.01.1991
(51) Int. Cl.: C07D 261/08

(54) **PROCESS FOR PRODUCING ISOXAZOLE DERIVATIVE**
VERFAHREN ZUR HERSTELLUNG EINES ISOXAZOLDERIVATS
PROCEDE DE PRODUCTION DE DERIVE D'ISOXAZOLE

(30) Priority: 24.01.1990 JP 14239/90
(43) Date of publication of application: 08.01.1992
(73) Proprietor: TAIHO PHARMACEUTICAL COMPANY LIMITED, Chiyoda-ku, Tokyo-to 101 (JP)
(72) Inventor: TANAKA, Motoaki, Tokorozawa-shi, Saitama-ken 359 (JP); HAGIWARA, Yuichi, Iruma-shi, Saitama-ken 358 (JP); KAJITANI, Makoto, Iruma-gun, Saitama-ken 350-12 (JP); YASUMOTO, Mitsugi, Honjo-shi, Saitama-ken 367 (JP)
(74) Representative: Barz, Peter, Dr.
(86) International application number: JP9100064
(87) International publication number: WO9111443

(56) References cited:
- EP-A- 0 026 928
- JP-A- 6 075 471

## Description

The present invention relates to a process for industrially advantageously producing isoxazole derivatives represented by the formula 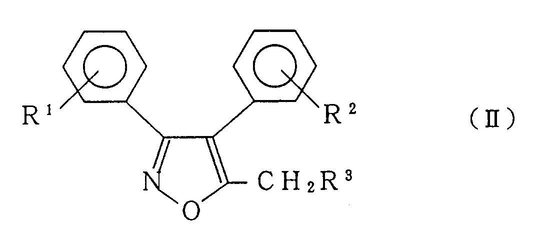
wherein R¹ and R² are the same or different and are each a hydrogen atom or lower C₁₋₆ alkoxyl, and R³ is cyano or alkoxycarbonyl.

The isoxazole derivatives to be produced by the process of the present invention are useful as intermediates for preparing (3,4-diarylisoxazol-5-yl)acetic acid derivatives which are represented by the formula 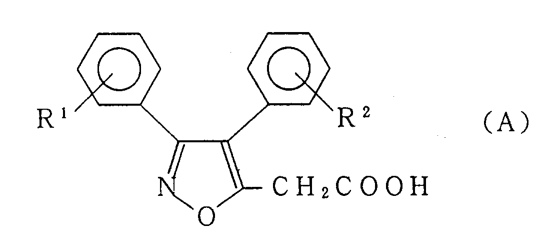
wherein R¹ and R² are the same or different and are each a hydrogen atom or lower C₁₋₆ alkoxyl, and which are useful as anti-inflammatory agents, analgesics and antipyretics.

Among the isoxazole derivatives represented by the formula (II) , the compounds wherein R³ is cyano are prepared by the known process which is disclosed in JP-A-75471/1985. This process comprises reacting 3,4-diaryl-5-methylisoxazole with a halogenating agent and then with a cyanogenation agent. The compounds of the formula wherein R³ is alkoxycarbonyl are novel compounds.

An object of the present invention is to provide a novel and preferred process, which is entirely different from the conventional process, for producing isoxazole derivatives represented by the formula (II) and useful as intermediates for preparing the compounds (A).

### Disclosure of the invention :

The present invention provides a process for producing isoxazole derivatives represented by the formula 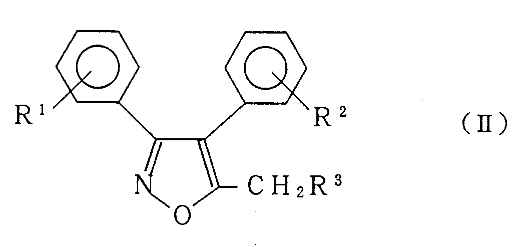
wherein R¹ and R² are the same or different and are each a hydrogen atom or lower C₁₋₆ alkoxyl, and R³ is cyano or alkoxycarbonyl, the process being characterized by oxidizing an α ,β -unsaturated ketoxime derivative represented by the formula 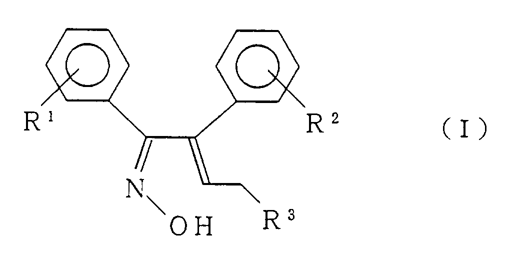
wherein R¹, R² and R³ are as defined above.

According to the present invention, preferred examples of lower alkoxyl groups represented by R¹ and R² are straight-chain or branched-chain alkoxyl groups having 1 to 6 carbon atoms, such as methoxy, ethoxy, n-propoxy, isopropoxy, n-butoxy, isobutoxy, sec-butoxy, tert-butoxy, pentyloxy and hexyloxy. Examples of alkoxycarbonyl groups represented by R³ are straight-chain or branched-chain alkoxycarbonyl groups having 2 to 7 carbon atoms, such as methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl, pentyloxycarbonyl and hexyloxycarbonyl.

The isoxazole derivatives to be produced by the process of the invention are useful as intermediates for preparing (3,4-diarylisoxazol-5-yl)acetic acid derivatives which are useful as anti-inflammatory agents, analgesics and antipyretics and which are represented by the formula 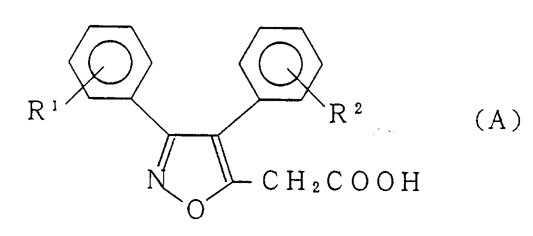
wherein R¹ and R² are the same or different and are each a hydrogen atom or lower alkoxyl.

The compound (I) for use in the present invention is prepared, for example, in accordance with the following reaction scheme. 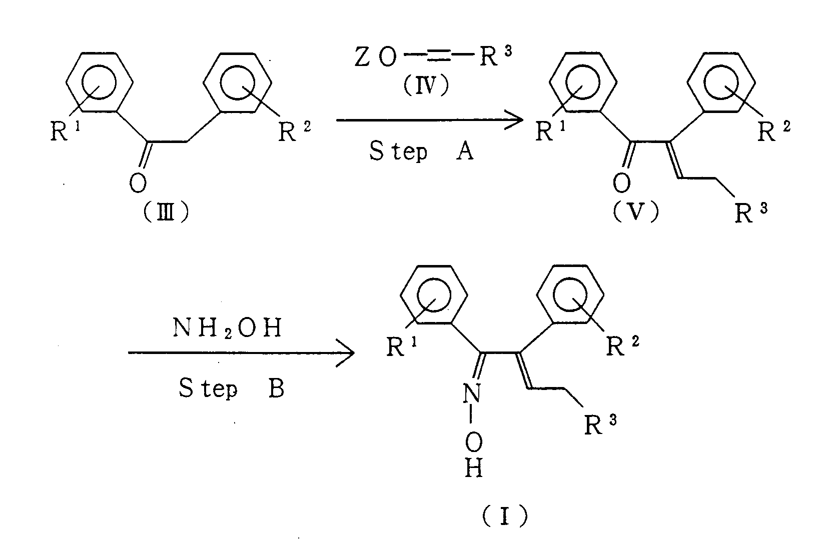
wherein R¹, R² and R³ are as defined above, and Z is lower alkyl.

Examples of lower alkyl groups represented by Z in the above scheme are straight-chain or branched-chain alkyl groups having 1 to 6 carbon atoms, such as methyl, ethyl, n-propyl, isopropyl, n-butyl, sec-butyl, tert-butyl, pentyl and hexyl.

More specifically, the steps represented by the above reaction scheme are performed in the following manner.

### Step A

A deoxybenzoin derivative represented by the formula (III) is reacted with an alkoxyacrylonitrile or alkoxyacrylic acid derivative represented by the formula (IV) in a suitable solvent in the presence of a base to obtain a compound of the formula (V). The solvent is not limited specifically insofar as it does not participate in the reaction. Examples of useful solvents are various organic solvents including methanol, ethanol, tert-butanol and like alcohols, tetrahydrofuran, dioxane and like ethers, benzene, toluene, xylene and like aromatic hydrocarbons, carbon tetrachloride, chloroform, dichloromethane and like hydrocarbon halides, acetonitrile, pyridine, dimethylformamide. These solvents can be used singly or in admixture. Examples of useful bases are sodium hydroxide, sodium methoxide, potassium tert-butoxide, butyl lithium and like alkali bases, triethylamine, dimethylaminopyridine and like organic bases. For the reaction, it is desirable to use 1 to 3 moles of the compound of the formula (IV) per mole of the compound of the formula (III), and 0.1 to 3 moles of the base per mole of the compound of the formula (III). The reaction is conducted at a temperature of up to 200 °C, preferably from 0 °C approximately to the boiling point of the solvent. The reaction usually takes about 0.5 to about 20 hours for completion.

### Step B

The compound represented by the formula (V ) and obtained by step A is reacted with hydroxylamine or a salt thereof in a suitable solvent to thereby obtain a compound represented by the formula (I). The salt of hydroxylamine to be used for the reaction is not limited specifically and is, for example, the hydrochloric acid salt or sulfuric acid salt. The solvent is not limited specifically insofar as it does not participate in the reaction. Examples of useful solvents are various organic solvents including methanol, ethanol, tert-butanol and like alcohols, tetrahydrofuran, dioxane and like ethers, benzene, toluene, xylene and like aromatic hydrocarbons, carbon tetrachloride, chloroform, dichloromethane and like hydrocarbon halides, acetonitrile, pyridine, dimethylformamide. These solvents can be used singly or in admixture. For the reaction, it is desirable to use 1 to 10 moles of hydroxylamine or a salt thereof per mole of the compound of the formula (IV). The reaction is conducted at a temperature of 0 to 200 °C, preferably from 40 °C approximately to the boiling point of the solvent. The completion of the reaction usually takes about 1 to about 30 hours.

The process of the invention for producing an isoxazole derivative represented by the formula (II) is characterized by oxidizing the compound of the formula (I) obtained according to the above reaction scheme. More specifically, the present invention resides in reacting the compound of the formula (I) with an oxidizing agent in a suitable solvent or in the absence of any solvent.

The oxidation process to be employed in the present invention is, for example, a process disclosed in "Lectures on New Experimental Chemistry," Vol.15, I -1, I -2, "Oxidation and Reduction," edited by the Chemical Society of Japan, published by Maruzen Co., Ltd. Examples of useful processes are a process using an oxidizing reagent such as potassium permanganate, manganese dioxide, potassium periodate, sodium periodate, ruthenium tetroxide or like oxide, lead tetracetate, mercury acetate, iron (III) chloride, potassium hexacyanoferrate (III) or like metal salt, hydrogen peroxide solution, peracetic acid or like peroxide, an autoxidation process using air or oxygen, an organic electrolytic oxidation process utilizing anodic oxidation.

For the reaction wherein the oxidizing reagent is used, it is desirable to use 0.2 to 10 moles of the reagent per mole of the compound of the formula (I). The solvent is not limited specifically insofar as it does not participate in the reaction. Examples of useful solvents are various organic solvents such as dichloromethane, chloroform, carbon tetrachloride and like hydrocarbon halides, benzene, toluene and like aromatic hydrocarbons, methanol, ethanol and like alcohols, diethyl ether, tetrahydrofuran and like ethers, acetone, hexane, acetic acid. These solvents can be used singly, in admixture or as admixed with water. The reaction temperature is -20 to 100 °C, preferably 5 to 70 °C. The completion of the reaction usually takes about 5 minutes to about 10 hours. When required, the reaction may be conducted with addition of an acid or base, or in a solvent mixture including a buffer.

The autoxidation process and the organic electrolytic oxidation process are conducted by passing air, oxygen or current through the reaction system in a suitable solvent. The solvent is not limited specifically insofar as it does not participate in the reaction. Examples of useful solvents are various organic solvents such as dichloromethane, chloroform, carbon tetrachloride and like hydrocarbon halides, benzene, toluene and like aromatic hydrocarbons, methanol, ethanol and like alcohols, diethyl ether, tetrahydrofuran and like ethers, acetone, hexane and acetic acid. These solvents can be used singly, in admixture, or as mixed with water. The reaction temperature is -20 to 100 °C, preferably 5 to 70 °C. The completion of the reaction usually takes about 5 minutes to about 24 hours. It is known that the reaction proceeds generally efficiently in the presence of a catalyst. Preferably, the catalyst is used in an amount of 1 × 10⁻⁵ to 10 moles per mole of the compound of the formula (I). Although the catalyst is not limited specifically, examples of useful catalysts are metals such as cobalt, rhodium, palladium, copper, cerium and ruthenium, or salts, oxides, complexes or like compounds of such metals. When required, the reaction may be conducted with addition of an acid or base, or in a solvent mixture including a buffer.

The compound of the invention thus obtained can be isolated and purified by usual known methods, for example, by distillation, recrystallization or silica gel column chromatography.

The isoxazole compound represented by the formula (II) and prepared by the above process is subjected, as isolated or as it is without isolation, to solvolysis or to hydrolysis in the presence of an acid or base, whereby a (3,4-diarylisoxazol-5-yl)acetic acid derivative represented by the formula (A) and having anti-inflammatory and analgesic activities can be derived from the compound of the invention. The solvolysis or hydrolysis can be conducted by solvolysis process disclosed in JP-A-75471/1985 or by the hydrolysis process generally employed in the art concerned. Generally used as the acid is an inorganic acid such as hydrochloric acid, sulfuric acid or nitric acid, or as the base is an inorganic base such as sodium hydroxide, potassium hydroxide or sodium carbonate.

The present invention will be described below in detail with reference to reference examples and examples.

### Reference Example 1

### Preparation of methyl 4,5-bis(4-methoxyphenyl)-5-oxo-3-pentenoate

To 430 mℓ of tert-butanol were added 128 g of deoxyanisoin, 67.3 g of potassium tert-butoxide and 116 g of methyl 3-methoxyacrylate, and the mixture was stirred at 70 °C for 3 hours. After the completion of reaction, the reaction mixture was allowed to stand at room temperature with addition of n-hexane. The product separating out was filtered off and dissolved with 1000 mℓ of ethyl acetate and 300 mℓ of 3N sulfuric acid. The organic layer was collected, washed with 3N sulfuric acid and a saturated aqueous solution of sodium chloride, and dried over anhydrous magnesium sulfate. The organic layer was concentrated at a reduced pressure, giving 153 g (yield 90 %) of the above-identified compound as an oily product.

An NMR spectrum revealed that the compound was a mixture of isomers (about 6:4) due to a double bond. The mixture was recrystallized from hexane-ethyl acetate as required, whereby one of the isomers was isolated in the form of white crystals.
Melting point 101∼ 103 °C
IR absorption spectrum (KBr)
ν max(cm⁻¹) 1732, 1640, 1600
NMR spectrum (CDCℓ₃) δ (ppm)
3.31(2H, d), 3.72(3H, s), 3.80(3H, s), 3.85(3H, s), 6.37(1H, t), 6.90(4H, d), 7.23(2H, d), 7.89(2H, d)
The mother liquor further gave the other isomer of the compound in the form of an oily product.
IR absorption spectrum (KBr)
ν max(cm⁻¹) 1732, 1662, 1596
NMR spectrum (CDCℓ₃) δ (ppm)
3.15(2H, d), 3.65(3H, s), 3.77(3H, s), 3.83(3H, s), 6.30(1H, t), 6.6∼ 7.1(4H, m), 7.30(2H, d), 7.92(2H, d)

### Reference Example 2

### Preparation of 4,5-bis(4-methoxyphenyl)-5-oxo-3-pentenenitrile

The identified compound was obtained as an oily product by conducting the same reaction as in Reference Example 1 with the exception of using 3-methoxyacrylonitrile instead of methyl 3-methoxyacrylate.
IR absorption spectrum (NaCℓ)
ν max(cm⁻¹) 2250, 1660, 1606
NMR spectrum (CDCℓ₃) δ (ppm)
3.17(2H, d), 3.78(3H, s), 3.85(3H, s), 6.03(3H, t), 6.7∼ 7.0(4H, m), 7.27(2H, d), 7.90(2H, d)

### Reference Example 3

### Preparation of methyl 5-hydroxyimino-4,5-bis(4-methoxyphenyl)-3-pentenoate

The isomer mixture of 4,5-bis(4-methoxyphenyl)-5-oxo-3-pentenoate (24.5 g) obtained in Reference Example 1 and 51.5 g of hydroxylamine hydrochloride was heated under reflux in a mixture of 650 mℓ of methanol and 72 mℓ of water for 23 hours. With the progress of reaction at this time, 0.9 equivalent weight of sodium hydrogencarbonate was added in divided portions to the reaction system. On completion of the reaction, the methanol was distilled off at a reduced pressure. The residue was dissolved with water and ethyl acetate, and the organic layer was collected, washed with a saturated aqueous solution of sodium chloride and dried over anhydrous magnesium sulfate. The organic layer was concentrated at a reduced pressure, and the residue was subjected to silica gel column chromatography (eluants: ethyl acetate-n-hexane) for separation and purification, affording 23 g (yield 90 %) of the above-identified compound as an oily product.
IR absorption spectrum (NaCℓ)
ν max(cm⁻¹) 1732, 1608
NMR spectrum (CDCℓ₃) δ (ppm)
3.1∼ 3.2(2H, m), 3.65(3H, s), 3.76(3H, s), 3.77(3H, s), 6.48(1H, t), 6.81(4H, d), 7.35(2H, d), 7.58(4H, d), 8.72(1H, bs)

### Reference Example 4

### Preparation of 5-hydroxyimino-4,5-bis(4-methoxyphenyl)-3-pentenenitrile

The identified compound was prepared as an oily product by conducting the same reaction as in Reference Example 3 with the exception of using 4,5-bis(4-methoxyphenyl)-5-oxo-3-pentenenitrile in place of 4,5-bis(4-methoxyphenyl)-5-oxo-3-pentenoate.
IR spectrum (NaCℓ)
ν max(cm⁻¹) 2252, 1596
NMR spectrum (CDCℓ₃) δ (ppm)
3.12, 3.15(2H, dd), 3.77(3H, s), 3.78(3H, s), 6.18(1H, t), 6.84(4H, d), 7.32(2H, d), 7.55(2H, d), 8.46(1H, bs)

### Example 1

### Preparation of 5-methoxycarbonylmethyl-3,4-bis(4-methoxyphenyl)isoxazole (II a)

A 3.7 g quantity of methyl 5-hydroxyimino-4,5-bis(4-methoxyphenyl)-3-pentenoate was heated at 60 °C with stirring for 24 hours in 40 mℓ of acetic acid in the presence of 0.4 g of cobalt acetate tetrahydrate while passing air through the mixture. After addition of 3N sulfuric acid, the reaction mixture was subjected to extraction with ethyl acetate, and the organic layer was washed with a saturated solution of potassium carbonate and then with a saturated aqueous solution of sodium chloride and dried over anhydrous magnesium sulfate. The organic layer was concentrated at a reduced pressure, and the residue was subjected to silica gel column chromatography (eluants: ethyl acetate-n-hexane) for separation and purification, giving 3.3 g (yield 90 % ) of the above-identified compound as a while solid product.
Melting point 67∼ 68 °C
IR absorption spectrum (KBr)
ν max(cm⁻¹) 1730
NMR spectrum (CDCℓ₃) δ (ppm)
3.73(3H, s), 3.77(2H, s), 3.79(3H, s), 3.82(3H, s), 6.83(2H, d), 6.90(2H, d), 7.15(2H d), 7.40(2H, d)
Mass spectrum
M⁺ (m/z) 353

### Example 2

### Preparation of 5-cyanomethyl-3,4-bis(4-methoxyphenyl)isoxazole (II b)

The identified compound was obtained as a white solid product (yield 80 % ) in the same manner as in Example 1 with the exception of using 5-hydroxyimino-4,5-bis(4-methoxyphenyl)-3-pentenenitrile in place of methyl 5-hydroxyimino-4,5-bis(4-methoxyphenyl)-3-pentenoate.
Melting point 103∼ 104 °C
IR absorption spectrum (KBr)
ν max(cm⁻¹) 2264
NMR spectrum (CDCℓ₃) δ (ppm)
3.80(3H, s), 3.83(2H, s), 3.85(3H, s), 6.8∼ 7.5(8H, m)
Mass spectrum
M⁺ (m/z) 320
A 1.77 g quantity of the 5-methoxycarbonylmethyl-3,4-bis(4-methoxyphenyl)isoxazole (II a) obtained in Example 1 was added to 15 mℓ of 2 % aqueous solution of sodium hydroxide, followed by stirring at 40 °C overnight. After the completion of reaction, the reaction mixture was washed with ether twice. While cooling the mixture with ice, 5 mℓ of 10 % hydrochloric acid was subsequently added thereto, followed by extraction with ethyl acetate, then washing with a saturated aqueous solution of sodium chloride and thereafter drying over anhydrous magnesium sulfate. The organic layer was concentrated at a reduced pressure, giving 3,4-bis(4-methoxyphenyl)-isoxazol-5-acetic acid as a white solid product (melting at 147∼ 148 °C).

### Example 3

### Preparation of 5-methoxycarbonylmethyl-3,4-bis(4-methoxyphenyl)isoxazole (II a)

A 1.75 g (5 mmols) quantity of methyl 5-hydroxyimino-4,5-bis(4-methoxyphenyl)-3-pentenoate was dissolved in 8.5 mℓ of dichloromethane and 4 mℓ of acetic acid, then 0.79 g of potassium permanganate was slowly added to the solution at room temperature, and the mixture was stirred for 4 hours. After the completion of reaction, a hydrogen peroxide solution was added to the reaction mixture until the mixture became transparent. The mixture was diluted with 10 mℓ of dichloromethane, subsequently washed with water, with sodium hydrogencarbonate and with a saturated aqueous solution of sodium chloride successively, and dried over anhydrous magnesium sulfate. The dried product was subjected to silica gel column chromatography (eluants: ethyl acetate-n-hexane) for separation and purification, affording 10.2 g (yield 60 % ) of the above-identified compound as a white solid product.

The melting point, IR absorption spectrum and NMR spectrum of the product coincided with those of the compound obtained in Example 1.

### Example 4

### Preparation of 5-methoxycarbonylmethyl-3,4-bis(4-methoxyphenyl)isoxazole (II a)

A 1.2 g (3.38 mmols) quantity of methyl 5-hydroxyimino-4,5-bis(4-methoxyphenyl)-3-pentenoate was dissolved in 19 mℓ of acetic acid, and the solution was added dropwise to a suspension composed of 0.44 g (5.1 mmols) of manganese dioxide and 5 mℓ of acetic acid at 60 °C. After the completion of addition, the mixture was stirred at 60 °C for one hour. After the completion of reaction, hydrogen peroxide was added to the reaction mixture to decompose excess manganese dioxide. The same procedure as in Example 3 was thereafter repeated to obtain 0.78 g (yield 65 % ) of the above-identified product.

The melting point, IR absorption spectrum and NMR spectrum of the product coincided with those of the compound obtained in Example 1.

The isoxazole derivative produced by the process of the invention is useful as an intermediate for preparing a (3,4-diarylisoxazol-5-yl)acetic acid derivative which is useful as an anti-inflammatory agent, analgesic and antipyretic and which is represented by the formula 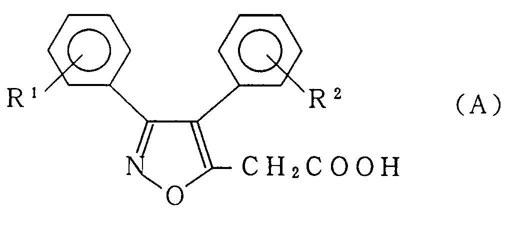
wherein R¹ and R² are the same or different and are each a hydrogen atom or lower C₁₋₆ alkoxyl.

## Claims

1. A process for producing isoxazole derivatives represented by the formula 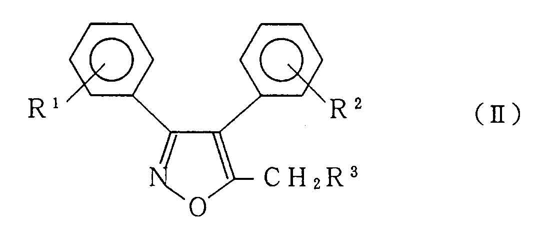 wherein R¹ and R² are the same or different and are each a hydrogen atom or lower C₁₋₆ alkoxyl, and R³ is cyano or alkoxycarbonyl, the process being characterized by oxidizing an α ,β -unsaturated ketoxime derivative represented by the formula 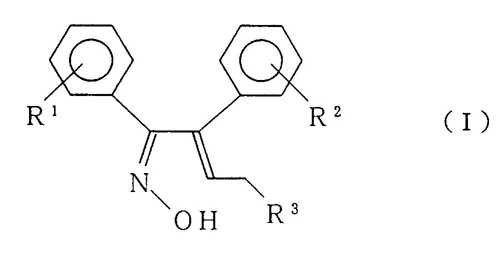 wherein R¹, R² and R³ are as defined above.

2. A process for producing isoxazole derivatives as defined in claim 1 wherein R¹ and R² are each a lower C₁₋₆ alkoxyl group.

3. A process for producing isoxazole derivatives as defined in claim 1 wherein the oxidation process is conducted by a process using an oxidizing reagent selected from potassium permanganate, manganese dioxide, potassium periodate, sodium periodate, ruthenium tetroxide, lead tetracetate, mercury acetate, iron (III) chloride, potassium hexacyanoferrate (III), hydrogen peroxide solution or peracetic acid, an autoxidation process using air or oxygen, or an organic electrolytic oxidation process utilizing anodic oxidation.

## Patentansprüche

1. Verfahren zur Herstellung von Isoxazol-Derivaten der Formel 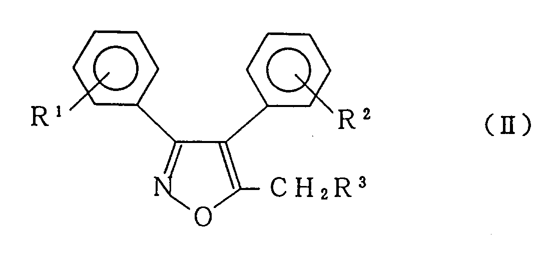 worin R¹ und R² gleich oder verschieden sind und jeweils ein Wasserstoffatom oder C₁₋₆-Niederalkoxyl darstellen, und R³ Cyano oder Alkoxycarbonyl ist, wobei das Verfahren gekennzeichnet ist durch Oxidieren eines α,β-ungesättigten Ketoxim-Derivats der Formel 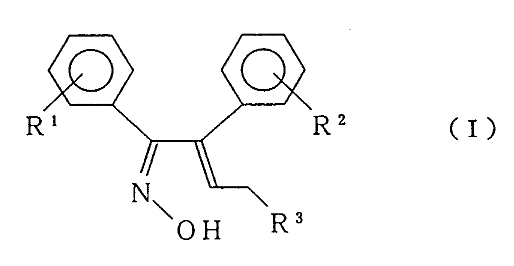 worin R¹, R² und R³ wie oben definiert sind.

2. Verfahren zur Herstellung von Isoxazol-Derivaten nach Anspruch 1, worin R¹ und R² jeweils eine C₁₋₆-Niederalkoxylgruppe sind.

3. Verfahren zur Herstellung von Isoxazol-Derivaten nach Anspruch 1, worin das Oxidationsverfahren durchgeführt wird durch
ein Verfahren unter Verwendung eines Oxidationsmittels, das aus Kaliumpermanganat, Mangandioxid, Kaliumperiodat, Natriumperiodat, Rutheniumtetroxid, Bleitetraacetat, Quecksilberacetat, Eisen(III)chlorid, Kaliumhexacyanoferrat (III), Wasserstoffperoxidlösung oder Peressigsäure ausgewählt ist,
ein Autooxidationsverfahren unter Verwendung von Luft oder Sauerstoff oder
ein organisches elektrolytisches Oxidationsverfahren mittels anodischer Oxidation.

## Revendications

1. Procédé pour la production de dérivés d'isoxazole représentés par la formule: 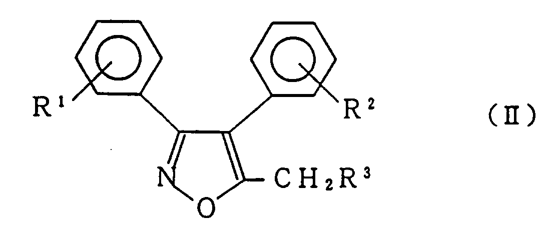 dans laquelle R¹ et R² sont identiques ou différents, et sont chacun, un atome d'hydrogène ou un groupe alcoxyle C₁₋₆ inférieur, et R³ est un groupe cyano ou alcoxycarbonyle, le procédé étant caractérisé par l'oxydation d'un dérivé cétoxime α,β-insaturée, représenté par la formule: 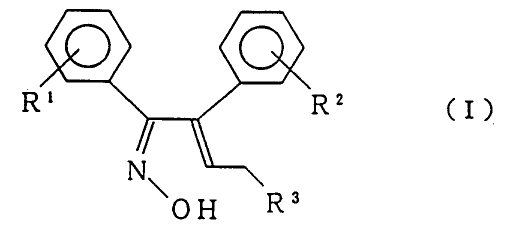 dans laquelle R¹, R², et R³ sont tels que définis plus haut.

2. Procédé pour la production de dérivés d'isoxazole selon la revendication 1, dans lequel R¹ et R² sont chacun, un groupe alcoxyle C₁₋₆ inférieur.

3. Procédé pour la production de dérivés d'isoxazole selon la revendication 1, dans lequel le procédé d'oxydation est conduit selon un processus utilisant un réactif oxydant choisi parmi le permanganate de potassium, le dioxyde de manganèse, le periodate de potassium, le periodate de sodium, le tétra-oxyde de ruthénium, le tétra-acétate de plomb, l'acétate de mercure, le chlorure de fer (III), l'hexacyanoferrate (III) de potassium, le peroxyde d'hydrogène en solution, ou l'acide peracétique, selon un processus d'auto-oxydation utilisant l'air ou l'oxygène ou selon un processus d'oxydation électrolytique organique utilisant l'oxydation anodique.
